(19) Europäisches Patentamt
European Patent Office
Office européen des brevets

(11) **EP 2 627 813 B1**

(12) **EUROPÄISCHE PATENTSCHRIFT**

(45) Veröffentlichungstag und Bekanntmachung des Hinweises auf die Patenterteilung:
**11.11.2015  Patentblatt 2015/46**

(21) Anmeldenummer: **11757150.5**

(22) Anmeldetag: **29.07.2011**

(51) Int Cl.:
*D06C 7/00* *(2006.01)*  *D01F 6/14* *(2006.01)*
*D01F 6/34* *(2006.01)*  *D01F 6/50* *(2006.01)*
*A61L 15/22* *(2006.01)*  *A61L 15/24* *(2006.01)*
*A61L 26/00* *(2006.01)*

(86) Internationale Anmeldenummer:
**PCT/EP2011/003808**

(87) Internationale Veröffentlichungsnummer:
**WO 2012/048768 (19.04.2012 Gazette 2012/16)**

(54) **HYDROGELIERENDE FASERN SOWIE FASERGEBILDE**

HYDROGEL FIBRES AND FIBROUS STRUCTURES

FIBRES HYDROGÉLIFIANTES AINSI QUE STRUCTURE FIBREUSE HYDROGÉLIFIANTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.10.2010  DE 102010048407**

(43) Veröffentlichungstag der Anmeldung:
**21.08.2013  Patentblatt 2013/34**

(73) Patentinhaber: **Carl Freudenberg KG**
**69469 Weinheim (DE)**

(72) Erfinder:
• **SCHMITZ, Wiebke**
**82131 Gauting (DE)**
• **SCHMIDT, Julia**
**45138 Essen (DE)**
• **SCHLESSELMANN, Bernd**
**69469 Weinheim (DE)**
• **SCHARFENBERGER, Gunter**
**67227 Frankenthal (DE)**

(56) Entgegenhaltungen:
**EP-A2- 0 745 708          WO-A1-2009/085679
US-A1- 2004 105 880**

• **KENNETH KAR HO WONG ET AL: "Effect of annealing on aqueous stability and elastic modulus of electrospun poly(vinyl alcohol) fibers", JOURNAL OF MATERIALS SCIENCE, KLUWER ACADEMIC PUBLISHERS, BO, Bd. 45, Nr. 9, 26. Januar 2010 (2010-01-26), Seiten 2456-2465, XP019790792, ISSN: 1573-4803**
• **SUZUKI A ET AL: "MECHANICAL PROPERTIES AND SUPERSTRUCTURE OF POLY(VINYL ALCOHOL) FIBERS ANNEALED UNDER EXTREMELY HIGH TENSION", KOBUNSHI RONBUNSHU (JAPANESE POLYMER SCIENCE AND TECHNOLOGY), SOCIETY OF POLYMER SCIENCE, TOKYO, JP, Bd. 51, Nr. 3, 1. Januar 1994 (1994-01-01) , Seiten 201-207, XP000445867, ISSN: 0386-2186**

EP 2 627 813 B1

**Beschreibung**

Technisches Gebiet

Die vorliegende Erfindung betrifft Fasern oder ein-, zwei- oder dreidimensionale Fasergebilde, sowie ein dazugehöriges Herstellungsverfahren. Des Weiteren betrifft die Erfindung die Verwendung von derartigen Fasern oder Fasergebilden als Trägermaterialien, Hygiene-, Kosmetikprodukte oder dergleichen und Wundverbände oder Wundauflagen, enthaltend derartige Fasern oder Fasergebilde.

Stand der Technik

Aus der WO 01/30407 A1 ist ein Verfahren zur Herstellung von Hydrogelen zur Verwendung als Wundauflagen bekannt, mit dem Verbrennungen oder andere Hautverletzungen behandelt werden können. Im Zuge des Verfahrens wird eine wässrige Lösung von Polyvinylalkohol, Agar-Agar und zumindest eines weiteren natürlichen Polymers zubereitet. Diese Lösung wird bei 70-80°C in Einweg-Plastikgefäßen abgefüllt und versiegelt. Nach Abkühlung auf Raumtemperatur werden die in den Einweg-Plastikgefäßen abgefüllten Proben bestrahlt und damit sterilisiert.

In der WO 2005/103097 A1 sind Hydrogele beschrieben, die zumindest ein Polyvinylalkohol-Sternpolymer aufweisen. Dabei werden die Hydrogele durch wiederholtes Einfrieren und Auftauen einer wässrigen Lösung enthaltend zumindest ein Polyvinylalkohol- Sternpolymer und optional weitere Komponenten hergestellt. Des Weiteren können derartige Hydrogele durch Einwirkung ionisierender Strahlung auf eine wässrige Lösung enthaltend zumindest ein Polyvinylalkohol - Sternpolymer oder durch eine Reaktion eines Polyvinylalkohol - Sternpolymers in wässriger Lösung mit vernetzenden Reagenzien hergestellt werden.

Nachteilig an den derzeitig bekannten Methoden zur Herstellung von Hydrogelen, insbesondere für die Wundbehandlung, ist die aufwendige Herstellungsweise und die problematische Weiterverarbeitung der Hydrogele, sowie gegebenenfalls das Auftreten von chemischen Verunreinigungen in den zum Beispiel durch eine chemische Reaktion vernetzten Hydrogelen. Zudem besitzen Hydrogel-Filme im Gegensatz zu Fasern und Fasergebilden eine kleinere Oberfläche, wodurch sie eine geringere Absorptionskapazität für Wasser oder wässrige Lösungen aufweisen. Besonders bei der Verwendung von Polyvinylalkohol als Rohmaterial für Hydrogele ist darauf zu achten, dass der Polyvinylalkohol einen dementsprechend hohen Vernetzungsgrad aufweist, da sich sonst keine Hydrogele, sondern Lösungen des Polyvinylalkohols in dem dementsprechenden flüssigen Medium ausbilden. Wünschenswert ist demzufolge eine hohe Stabilität des Polyvinylalkohols gegenüber der Löslichkeit in Wasser oder in wässrigen Lösungen. Außerdem zeichnen sich gerade Polyvinylalkohol und Polyvinylalkohol-Copolymere durch eine hohe Biokompatibilität und Bioverträglichkeit aus, so dass ein zunehmender Bedarf an weiteren Ausführungsformen von Hydrogelen oder hydrogelierenden Materialien mit Polyvinylalkohol und/oder Polyvinylalkohol-Copolymeren besteht, die zudem kostengünstig und einfach herzustellen sind und eine unproblematische Weiterverarbeitung ermöglichen.

KENNETH KAR HO WONG et al:"Effect of annealing on aqueous stability and elastic modulus of electrospun poly(vinyl alcohol) fibers", JOURNAL OF MATERIALS SCIENCE, KLUWER ACADEMIC PUBLISHERS, BO, Bd. 45, Nr. 9, 26. Januar 2010 (2010-01-26), Seiten 2456-2465, beschreibt den Effekt einer Hitzebehandlung auf die Stabilität in wässrigen Lösungen und den Elastizitätsmodulus von elektroversponnenen Poly(vinyl)alkohol Fasern. Es wird beschrieben, daß mittels Elektrospinnen dreidimensionale Vliesstoffe aus Fasern, deren Durchmesser im Nanobereich liegt, hergestellt werden. Die elektroversponnenen Fasern werden in einem Vakuumofen bei verschiedenen Temperaturen und Behandlungszeiten unter Druck hitzebehandelt. Die elektroversponnenen Poly(vinylalkohol)lfasern werden durch die in dem Dokument beschriebene Wärmebehandlung mit einer erhöhten Wasserstabilität versehen.

SUZUKI A et al:"Mechanical Properties and superstructure of Poly(vinyl alcohol)fibers annealed under extremely high tension", Kobunshi Ronbunshu (Japanese Polymer Science and Technology), Society of Polymer Science, Tokyo, JP, Bd. 51, Nr. 3, 1. Januar 1994, Seiten 201-207, beschreibt die mechanischen Eigenschaften und die Superstruktur von Poly(vinyl)alkohol Fasern, welche unter extrem hoher Spannung getempert werden. Durch das Tempern bei extrem hohen Spannungen werden die Fasern mit einer hohen Festigkeit und Stabilität gegenüber Wasser bzw. wässrigen Lösungen versehen.

EP 0 745 708 A2 beschreibt Fasern auf Basis von Polyvinylalkohol, welche durch Verspinnen einer Lösung eines polyvinylalkoholbasierten Polymers, welches Ammoniumsulfat als Vernetzer enthält, Trocknung der Fasern, Verstrecken bei Temperaturen von 100° C bis 200° C und Vernetzen bei einer Temperatur von 210° C oder höher erhalten werden. Vermutlich durch den Einsatz von Ammoniumsulfat als Vernetzer bei Vernetzungstemperaturen von über 210° C werden Fasern erhalten, die eine sehr stark vernetzte Struktur aufweisen. Aus diesem Grund weisen die Fasern eine nur geringe Aufnahmekapazität für wässerige Medien auf.

Darstellung der Erfindung

Die vorliegende Erfindung beschäftigt sich deshalb mit der Aufgabe, für Fasern oder Fasergebilde, deren Verwendung, sowie ein zugehöriges Herstellungsverfahren und für Wundverbände oder Wundauflagen eine verbesserte oder zumindest eine alternative Ausführungsform anzugeben, die sich insbesondere durch eine vereinfachte, kostengünstige Herstellung auszeichnen und eine unproblematische Weiterverarbeitung und/oder Anwendung ermöglichen.

Erfindungsgemäß wird diese Aufgabe durch die Gegenstände der unabhängigen Ansprüche gelöst. Vorteilhafte Ausführungsformen sind Gegenstand der abhängigen Ansprüche.

Überraschenderweise konnte nun festgestellt werden, dass Fasern oder Fasergebilde, die Polyvinylalkohol oder Polyvinylalkohol-Copolymere enthalten, so durch Tempern behandelt werden können, dass sie gelierende, insbesondere hydrogelierende, Eigenschaften nach dem Tempern aufweisen und zudem mit einer hohen Stabilität zumindest gegenüber der Löslichkeit in Wasser oder wässrigen Lösungen ausgestattet sind. Zudem zeichnen sich die Fasern oder Fasergebilde durch eine besonders hohe Aufnahme an Wasser oder wässrigen Lösungen, insbesondere 0,9 prozentige, wässrige Natriumchlorid-Lösung (physiologische Kochsalzlösung) aus.

In einem ersten Aspekt der Erfindung werden somit Fasern oder Fasergebilde, ein-, zwei-oder dreidimensional, vorgeschlagen, die Fasern aus einem ersten Faserrohmaterial aufweisen, das unsubstituierten oder teilweise substituierten Polyvinylalkohol und/oder unsubstituierte oder teilweise und substituierte Polyvinylalkohol-Copolymere aufweist, wobei die Fasern durch Tempern vernetzt sind. Dabei sind die Fasern oder Fasergebilde gelierend, insbesondere hydrogelierend ausgebildet.

Vorteilhaft können durch das Tempern die Fasern oder Fasergebilde vernetzt werden, so dass zum einen die Fasern oder Fasergebilde nach dem Tempern eine höhere Stabilität zumindest gegenüber der Löslichkeit in Wasser aufweisen. Zum anderen erhalten die Fasern oder Fasergebilde durch das Tempern die Fähigkeit, mit Wasser oder wässrigen Lösungen, insbesondere mit 0,9 prozentiger Natriumchlorid-Lösung, ein stabiles Gel auszubilden. Zudem können durch das Tempern Verunreinigungen oder Rückstände, wie zum Beispiel Spinnhilfsmittel, Avivage, Lösungsmittel oder dergleichen, auf eine vernachlässigbare, fast nicht mehr nachweisbare Konzentration reduziert werden. Des Weiteren weisen derartige Fasern oder Fasergebilde eine hohe Aufnahmekapazität für Wasser, insbesondere für eine 0,9 prozentige, wässrige Natriumchlorid-Lösung auf. Somit lassen sich vorteilhaft toxikologisch unbedenkliche Fasern oder Fasergebilde, sowie daraus herstellbaren Gele, insbesondere Hydrogele, erzeugen. Dabei ist die Prozentangabe der Natriumchlorid-Lösung in Gew.-% angegeben.

Folglich ist die eingangs genannte Aufgabe gelöst.

Man versteht unter Fasern ein im Verhältnis zu seiner Länge dünnes und flexibles Gebilde. Fasern weisen einen geringen Durchmesser auf und können miteinander durch dementsprechende Verfestigungsverfahren zu Fasergebilden aufgebaut werden. Somit kann ein Fasergebilde mehrere Fasern aufweisen. Man kann zwischen ein-, zwei- und dreidimensionale Fasergebilden unterscheiden. Ein eindimensionales Fasergebilde hat im Vergleich zu seiner Länge eine geringe Breite und eine geringe Höhe. Ein zweidimensionales Fasergebilde hat im Vergleich zu seiner Länge und Breite eine geringe Höhe. Unter dreidimensionalen Fasergebilden sind Fasergebilde zu verstehen, die mehrere Lagen von zweidimensionalen Fasergebilden aufweisen. Dabei können die einzelnen Lagen des dreidimensionalen Fasergebildes durch nachfolgend beschriebene Verfestigungsverfahren oder anderweitig zueinander verbunden werden.

Natürlich vorkommende Naturfasern können als eindimensionale Fasergebilde bezeichnet werden. Aus gewaschenen und aufbereiteten Naturfasern lassen sich durch Garnspinnverfahren Naturfasergarne erhalten, die eindimensionale Fasergebilde darstellen können. Außerdem lassen sich aus synthetischen, natürlichen oder modifizierten natürlichen Polymeren mittels des Trocken- oder des Nassspinnverfahrens Filamente herstellen und mittels des Spinnvliesverfahrens Spinnvliese. Die Filamente können dabei als eindimensionale Fasergebilde angesehen werden, während die Spinnvliese zweidimensionale Fasergebilde darstellen können. Durch Schneiden und/oder Kräuseln der Filamente können Stapelfasern hergestellt werden, die als eindimensionale Fasergebilde eingeordnet werden können. Durch Garndrehung können aus Stapelfasern Stapelfasergarne hergestellt werden. Sie können als eindimensionale Fasergebilde verstanden werden. Aus Filamenten aufgebaute Garne können aus einem Filament (Monofilamentgarn) oder mehreren Filamenten (Multifilamentgarn) ausgebildet sein. Sie können ebenfalls als eindimensionale Fasergebilde angesehen werden. Mischgarne können durch Garnspinnen mehr als einer unterschiedlichen Stapelfaser bzw. Naturfaser hergestellt werden. Garne wie Naturfasergarne, Stapelfasergarne oder Filamentgarne oder Mischgarne, können mittels textiltechnischer Verfahren wie Weben, Stricken, Wirken, Sticken, Legen oder Nähen z.B. zu Geweben, Gestricken, Gelegen oder Gewirken weiterverarbeitet werden. Die Gewebe, Gestricke, Gelege bzw. Gewirke können als zweidimensionale Fasergebilde angesehen werden. Mittels vliestechnischer Verfahren wie Krempeln, dem Airlaidverfahren oder dem Nassvliesverfahren können aus Stapelfasern Stapelfaservliese oder Airlaidvliese bzw. Airlaids, oder Naßvliese hergestellt werden, die ebenfalls als zweidimensionale Fasergebilde angesehen werden können.

Unverfestigte Vliese, z.B. Stapelfaser- oder Spinnvliese, können durch Verfestigungsverfahren verfestigt werden. Es kann beispielsweise als Verfestigungsverfahren ein Kalandrieren angewendet werden. Dabei werden die unverfestigten Vliese zwischen Rollen geführt, wobei auf den Rollen angeordnete Verschweißflächen in den Vliesen zumindest teilweise die Vliese durchdringende Verschweißungen erzeugen. Werden punktförmige Verschweißungen erzeugt, dann wird das Verfestigungsverfahren als PS (point seal)-Verfestigungsverfahren bezeichnet. Es ist aber auch die Ausbildung von linienförmigen Verschweißungen oder vollflächigen Verschweißungen möglich. Als weiteres Verfestigungsverfahren kann eine Heißluftverfestigung im Durchlufttrockner angewendet werden, wobei bei diesem Verfahren Verfestigungen durch Verschmelzungen an den Berührungspunkten der Fasern erzeugt werden. Des Weiteren ist ebenfalls der Einsatz von Bindern oder Bindemitteln denkbar, wobei hierbei die Fasern über Brücken aus Bindern oder Bindemittel miteinander verbunden werden. Es können auch mechanische Verfestigungsverfahren zum Einsatz kommen, wie z.B. das Nadelverfestigungsverfahren, bei dem die Verfestigung mittels Nadeln vorgenommen wird und/oder das Wasserstrahlverfestigungsverfahren, bei dem Wasserstrahlen zum Einsatz kommen. Des Weiteren ist ebenfalls Walken oder Filzen oder dergleichen denkbar. Dabei kann auch eine Kombination von mehreren Verfestigungsverfahren angewendet werden. Bevorzugt werden das Nadelverfestigungsverfahren, das Wasserstrahlverfahren und/oder das PS-Verfestigungsverfahren angewendet.

Durch das Tempern werden die Fasern vernetzt. Somit können die Fasern selbst als auch die daraus herstellbaren Fasergebilde durch Tempern derartig verändert werden, dass sie eine höhere Stabilität hinsichtlich der Löslichkeit in Wasser, insbesondere in einer 0,9 prozentigen, wässrigen Natriumchlorid-Lösung aufweisen und zum anderen hervorragende Gelbildner darstellen.

Unter einem Gel ist dabei ein feindisperses System aus mindestens einer festen und einer flüssigen Phase zu verstehen, wobei die feste Phase ein dreidimensionales Netzwerk ausbildet, dessen Poren durch die flüssige Phase ausgefüllt sind. Beide Phasen durchdringen sich dabei vollständig und demzufolge kann ein Gel im Vergleich zu einem Schwamm die flüssige Phase stabiler gegenüber z.B. Druck speichern. Erfindungsgemäße Fasern oder Fasergebilde sind gelierend, insbesondere hydrogelierend, ausgebildet und weisen demzufolge ein hervorragendes Bindungsvermögen für dementsprechende flüssige Phasen auf. Sie werden bevorzugt trocken auf die Wunde aufgelegt und bilden mit dem Wundexudat stabile Gele aus und schaffen somit ein feuchtes Wundklima. Eine derartige feuchte Wundbehandlung kann den Heilungsprozess unterstützen.

Ebenfalls für die feuchte Wundbehandlung können sie mit einer flüssigen Phase bestückt in gelierter Form eingesetzt werden. Bevorzugt wird dabei als flüssige Phase Wasser verwendet und besonders bevorzugt eine 0,9 prozentige, wässrige Natriumchlorid-Lösung, Ringer-Lösung oder wirkstoffhaltige Lösungen. Demzufolge ist unter gelierend die Fähigkeit zur Ausbildung eines Gels unter Aufnahme einer flüssigen Phase zu verstehen und unter hydrogelierend die Fähigkeit zur Ausbildung eines Hydrogels, das als flüssige Phase Wasser oder eine wässrige Lösung, besonders bevorzugt eine 0,9 prozentige, wässrige Natriumchlorid-Lösung, aufweist.

Als erstes Faserrohmaterial wird Polyvinylalkohol verwendet. Es kann auch zumindest ein Polyvinylalkohol-Copolymer verwendet werden. Im Falle eines Polyvinylalkohol-Copolymers kann beispielsweise Polyethylen-Vinylalkohol zum Einsatz kommen.

Werden Polyvinylalkohol-Copolymere als erstes Faserrohmaterial verwendet, so können weitere physikalische wie auch chemische Eigenschaften gegebenenfalls gezielt in die Fasern eingebaut werden. So ist im Fall der Verwendung von beispielsweise Polyethylen-Vinylalkohol die Anzahl an OH-Gruppen reduziert ausgebildet. Es können aber auch andere funktionelle Gruppen in die Fasern mittels Copolymerisation eingebracht werden. Somit stehen durch die Polyvinylalkohol-Copolymere weitere erste Faserrohmaterialien zur Verfügung.

Bevorzugt wird Polyethylen-Vinylalkohol, Polyvinylalkohol-Styrol, Polyvinylalkohol-Vinylacetat, Polyvinylalkohol-Vinylpyrrolidon, Polyvinylalkohol-ethylenglykol und/oder Polyvinylalkohol, besonders bevorzugt Polyethylen-Vinylalkohol, Polyvinylalkohol-Vinylacetat, Polyvinylalkohol-Vinylpyrrolidon, Polyvinylalkohol-Vinylamin, Polyvinylalkohol-Acrylat, Polyvinylalkohol-Acrylamid, Polyvinylalkohol-ethylenglykol und/oder Polyvinylalkohol und ganz besonders bevorzugt Polyvinylalkohol als erstes Faserrohmaterial eingesetzt. Es können auch Blockcopolymere und/oder Pfropfcopolymere und/oder Block-Pfropfcopolymere, statistische oder alternierende Systeme und jegliche Mischungen untereinander als erstes Faserrohmaterial eingesetzt werden.

Sowohl das Polyvinylalkohol-Copolymer als auch der Polyvinylalkohol kann dabei unsubstituiert oder teilweise substituiert zum Einsatz kommen. Im Falle der teilweisen Substitution ist die Substitution der OH-Gruppen durch -O(C=O)-R oder -OR denkbar, wobei R jeweils unabhängig voneinander steht für einen $C_1$-$C_4$ Alkylrest. Dabei versteht man unter einem $C_1$-$C_4$ Alkylrest Methyl, Ethyl, Propyl, iso-Propyl, 1-Butyl, 2-Butyl, oder tert.-Butyl.

Bevorzugt kommt dabei das Polyvinylalkohol-Copolymer oder der Polyvinylalkohol zumindest teilweise mit -O(C=O)-R oder -OR substituiert oder unsubstituiert, besonders bevorzugt mit -O(C=O)-R, insbesondere wenn R steht für Ethyl, substituiert oder unsubstituiert und ganz besonders bevorzugt unsubstituiert zum Einsatz.

Des Weiteren kann das erste Faserrohmaterial als Polymer-Blend ausgebildet sein. Dabei versteht man unter einem Polymer-Blend eine physikalische Mischung von mindestens zwei Polymeren aus der Schmelze oder aus Lösung.

Vorteilhaft weist der daraus resultierende Polymer-Blend im Vergleich zu den eingesetzten Polymeren unterschiedliche physikalische Eigenschaften und gegebenenfalls auch chemische Eigenschaften auf. Üblicherweise sind dabei die Eigenschaften des Polymer-Blends eine Summe der Eigenschaften der verwendeten Polymere. Somit kann durch den Einsatz von Polymer-Blends eine Auswahl an ersten Faserrohmaterialien weiter vergrößert werden.

Dabei können zur Ausbildung eines derartigen Polymer-Blends gelierende weitere Polymere, wie z.B. Alginate, Celluloseether, wie Carboxymethylcellulosen, Methyl-, Ethylcellulosen,, Hydroxymethylcellulosen, Hydroxyethylcellulosen, Hydroxyalkylmethylcellulosen, Hydroxypropylcellulosen, Celluloseester, wie Celluloseacetat, oxidierte Cellulosen, bakterielle Cellulosen, Cellulosecarbonate, Gelatinen, Kollagene, Stärken, Hyaluronsäuren, Pektine, Agar, Polyacrylate, Polyvinylamine, Polyvinylacetate, Polyethylenglycole, Potyethylenoxide, Polyvinylpyrrolidone, Polyurethane oder nicht-gelierende weitere Polymere, wie z.B. Polyolefine, Cellulose, Cellulosederivate, regenerierte Cellulose wie Viskose, Polyamide, Polyacrylnitrile, Polyvinylchloride, Chitosane, Polylactide, Polyglykolide, Polyesteramide, Polcaprolactone, Polyhexamethylenterephthalate, Polyhydroxybutyrate, Polyhydroxyvalerate oder Polyester eingesetzt werden.

Die oben aufgeführten Blends können als Homopolymere oder Copolymere eingesetzt werden. Es können auch Blockcopolymere und/oder Pfropfcopolymere und/oder Block-Pfropfcopolymere, statistische oder alternierende Systeme und jegliche Mischungen untereinander eingesetzt werden.

Dabei versteht man unter Alginate die Salze der Alginsäure, einem natürlichen in Algen vorkommenden Polymer, der beiden Uronsäuren α-L-Guluronsäure und β-D-Mannuronsäure, die 1,4-glycosidisch verknüpft sind. Dabei wird von dem Begriff Alginat E401, E402, E403, E404 und E405 (PGA) umfasst. Von dem Begriff Polyolefine wird PE, PB, PIB und PP umfasst. Von dem Begriff Polyamiden werden PA6, PA6.6, PA6/6.6, PA6.10, PA6.12 PA69, PA612, PA11, PA12, PA46, PA1212 und PA6/12 umfasst. Von dem Begriff Cellulose wird auch regenerierte Cellulose wie Viskose, sowie Cellulosederivate und chemisch und/oder physikalisch modifizierte Cellulose umfasst. Von dem Begriff Polyester werden PBT, BC, PET, PEN und UP umfasst.

Bevorzugt ist die Verwendung von gelierenden weiteren Polymeren, unsubstituierten oder teilweise substituierten Polyvinylalkohol-Copolymeren, wie Polyethylen-Vinylalkohol, Polyvinylalkohol-Styrol, Polyvinylalkohol-Vinylacetat, Polyvinylalkohol-Vinylpyrrolidon, Polyvinylalkohol-Ethylenglykol, Polyvinylalkohol-Vinylamin, Polyvinylalkohol-Acrylat, Polyvinylalkohol-Acrylamid und/oder Polyvinylalkohol, besonders bevorzugt die Verwendung von unsubstituierten oder teilweise substituierten Polyvinylalkohol-Copolymeren, wie Polyethylen-Vinylalkohol, Polyvinylalkohol-Vinylacetat, Polyvinylalkohol-Vinylpyrrolidon, Polyvinylalkohol-Vinylamin, Polyvinylalkohol-Acrylat, Polyvinylalkohol-Acrylamid, Polyvinylalkohol-Ethylenglykol und/oder Polyvinylalkohol und ganz besonders bevorzugt die Verwendung von unsubstituierten Polyvinylalkohol-Copolymeren und/oder Polyvinylalkohol zur Herstellung des als Polymer-Blends ausgebildeten ersten Faserrohmaterials, enthaltend zumindest Polyvinylalkohol und/oder zumindest ein Polyvinylalkohol-Copolymer. Es können auch Blockcopolymere und/oder Pfropfcopolymere und/oder Block-Pfropfcopolymere, statistische oder alternierende Systeme und jegliche Mischungen untereinander eingesetzt werden.

Außerdem können die Fasern oder Fasergebilde zusätzlich weitere Fasern aus zumindest einem zweiten Faserrohmaterial aufweisen. Dabei kann das zumindest eine zweite Faserrohmaterialien nicht-gelierend oder gelierend ausgebildet sein. Somit können als weitere Fasern nicht-gelierende oder gelierende Fasern eingesetzt werden.

Vorteilhaft kann durch den Einsatz von weiteren Fasern ein gewünschtes Verhalten der Fasern oder Fasergebilde gezielt verbessert werden. So ist es denkbar durch den Einsatz der weiteren Fasern die Stabilität der Fasern oder Fasergebilde zu verbessern, in dem die weiteren Faser als, insbesondere gegenüber mechanischen Beanspruchungen, stabilisierendes Gerüst wirken.

Als nicht-gelierendes, zweites Faserrohmaterial können Polyolefine, Cellulose, Cellulosederivate, regenerierte Cellulose, wie Viskose, Polyamide, Polyacrylnitrile, Chitosane, Elastane, Polyvinylchloride, Polylactide, Polyglykolide, Polyesteramide, Polycaprolactone, Polyhexamethylenterephthalate, Polyhydroxybutyrate, Polyhydroxyvalerate, tierische und/oder pflanzliche Naturfasern und/oder Polyester und als gelierendes, zweites Faserrohmaterial Alginate, Celluloseether, wie Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie Celluloseacetat, oxidierte Cellulosen, bakterielle Cellulosen, Cellulosecarbonate, Gelatinen, Kollagene, Stärken, Hyaluronsäuren, Pektine, Agar, Polyvinylamine, Polyvinylacetate, Polyethylenglycole, Polyethylenoxide, Polyvinylpyrrolidone, Polyurethane und/oder Polyacrylate eingesetzt werden. Die aufgeführten zweiten Faserrohmaterialien können sowohl als Homopolymere als auch als Copolymere eingesetzt werden. Es können auch Blockcopolymere und/oder Pfropfcopolymere und /oder Block-Pfropfcopolymere, statistische oder alternierende Systeme und jegliche Mischungen untereinander eingesetzt werden. Es ist auch der gleichzeitige Einsatz von gelierenden und nicht-gelierenden weiteren Fasern möglich. Bevorzugt ist dabei die Verwendung von weiteren Fasern aus einem zweiten, nicht-gelierenden Faserrohmaterial, besonders bevorzugt aus Polyamid und/oder Polyester und ganz besonders bevorzugt aus Polyester.

Die weiteren Fasern können auch aus einem als Polymer-Blend ausgebildeten zweiten Faserrohmaterial hergestellt sein. Dabei ergeben sich für die weiteren Fasern die schon vorhergehend bei dem ersten Faserrohmaterial aufgezeigten Vorteile.

Als weitere Fasern können auch Bikomponentenfasern und/oder Mehrkomponentenfasern, gelierend oder nicht-gelierend, verwendet werden. Dabei können die Bikomponentenfasern und/oder Mehrkomponentenfasern mit geometrischen Formen wie "Core shell", "Side-by-Side", "Pie- oder Orangetype", "Matrix with fibrils" eingesetzt werden. Die Bikomponentenfasern und/oder Mehrkomponentenfasern können zur thermischen Verfestigung der Vliese genutzt werden. Bei Erwärmung dieser Fasern erfolgt eine thermische Bindung des Vlieses. Beispielsweise schmilzt bei einer Core shell-Faser der shell-Anteil und verfestigt somit das Vlies. Als Bikomponentenfasern und/oder Mehrkomponentenfasern können Fasern aus Polyethylen/Polypropylen, Polyethylen/Polyester, Co-Polyester/Polyethylenterephthalat, Polyamid6/ Polyamid6.6, Polybutylenterephthalat/Polyethylenterephthalat eingesetzt werden.

Vorteilhaft kann durch die Verwendung von zusätzlichen, nicht-gelierenden Fasern die Aufnahmekapazität von Wasser, insbesondere von einer 0,9 prozentigen Natriumchlorid-Lösung, im Vergleich zu Fasern oder Fasergebilden ohne zusätzliche, nicht-gelierende Fasern deutlich erhöht werden, da mittels der nicht- gelierenden Fasern ein Gel-Blocking-Effekt, der ab einer vorbestimmten Sättigung eine weitere Aufnahme von Wasser, insbesondere von einer 0,9 prozentigen Natriumchlorid-Lösung, verhindert, verringert werden kann. Zudem lassen sich die Fasern oder Fasergebilde durch die Verwendung von nicht- gelierende Fasern stärker mechanisch stabilisieren und zudem kann der Schrumpf der Fasern oder Fasergebilde durch Beimengen von nicht-gelierenden Fasern deutlich verringert werden.

Dabei kann der Schrumpf von zumindest zweidimensionalen Fasergebilden durch Ausstanzen von 10,0 cm x 10,0 cm (Fläche1) große Stücken und Eintauchen derselben in eine 0,9 %ige wässrige Natriumchlorid-Lösung bestimmt werden. Die ausgestanzten und getränkten Stücke werden aus der Lösung entnommen und für 2min abgetropft. Danach wird die Größe der Stücke abgemessen (Fläche 2). Der Schrumpf der Vliesstoffe kann dann nach folgender Formel berechnet werden:

$$Schrumpf \; [\%] = 100 - \frac{Fläche2 \; [cm^2]}{Fläche1 \; [cm^2]} * 100$$

Der Anteil an weiteren Fasern in den Fasern oder den Fasergebilden kann 1 bis 70 Gew.% betragen. Bevorzugt beträgt der Anteil 1 bis 65 Gew.%, besonders bevorzugt 5 bis 60 Gew.% und ganz besonders bevorzugt 10 bis 50 Gew.%.

Die weiteren Fasern können einen Fasertiter von 0,9 bis 10 dtex aufweisen. Bevorzugt werden sie mit einen Fasertiter von 1 bis 9 dtex eingesetzt, besonders bevorzugt mit einen Fasertiter von 1,5 bis 7 dtex und ganz besonders bevorzugt mit einen Fasertiter von 1,9 bis 4 dtex. Dabei ist unter dtex oder Dezitex das Gewicht in Gramm der Faser bei einer ggf. theoretischen Länge von 10.000 m zu verstehen.

Die weiteren Fasern können eine Länge von 30 bis 80 mm aufweisen. Bevorzugt werden sie mit einer Länge von 32 bis 76 mm eingesetzt, besonders bevorzugt mit einer Länge von 35 bis 74 mm und ganz besonders bevorzugt mit einer Länge von 38 bis 70 mm.

Wird das Airlaid-Verfahren angewendet, so können die weiteren Fasern eine Länge von 5 bis 20 mm aufweisen. Bevorzugt werden sie dabei mit einer Länge von 6 bis 19 mm eingesetzt, besonders bevorzugt mit einer Länge von 7 bis 18 mm und ganz besonders bevorzugt mit einer Länge von 8 bis 17 mm.

Des Weiteren können die Fasern oder die Fasergebilde zusätzlich Additive aufweisen. Dabei können als Additive pharmakologische Wirkstoffe oder Medikamente, wie Antibiotika, Analgetika, Antiinfektiva, entzündungshemmende Mittel, wundheilungsfördernde Mittel oder dergleichen, antimikrobielle, antibakterielle oder antivirale Agentien, blutstillende Mittel, Enzyme, Aminosäuren, Antioxidantien, Peptide und/oder Peptidsequenzen, Polysaccharide (z.B. Chitosan), Wachstumsfaktoren (z.B. Purine, Pyrimidine), lebende Zellen, β-Tricalciumphosphat, Hydroxyapatit, insbesondere speziell Hydroxyapatitnanopartikel, geruchsadsorbierende Additive wie Aktivkohle, Cyclodextrine, Metalle wie Silber, Gold, Kupfer, Zink, Kohlenstoffverbindungen, wie Aktivkohle, Graphit oder dergleichen und/oder Verarbeitungshilfsstoffe wie oberflächenaktive Substanzen, Netzmittel, Avivagen, Antistatika eingesetzt werden.

Durch den Einsatz von zumindest einem Additiv können die Fasern oder Fasergebilde zudem vorteilhaft mit weiteren physikalischen, chemischen sowie biologischen Eigenschaften ausgestattet werden. So ermöglicht beispielsweise eine Beschichtung der Fasern oder Fasergebilde mit Silber eine antibakterielle Wirkung der Fasern oder Fasergebilde.

Die Fasern oder Fasergebilde können eine besonders hohe Aufnahmekapazität für Wasser, wässrige Lösungen und Wundexudat aufweisen. Die Fasern oder Fasergebilde können eine Aufnahmekapazität für 0,9 prozentige Natriumchlorid-Lösung von 300 bis 3500 Gewichtsprozent aufweisen. Im Fall von Fasern und/oder eindimensionalen, sowie zweidimensionalen Fasergebilden liegt erfindungsgemäß eine Aufnahmekapazität für 0,9 prozentige Natriumchlorid-Lösung von 300 bis 3500 Gewichtsprozent vor, besonders bevorzugt von 400 bis 2500 Gewichtsprozent und ganz besonders bevorzugt von 500 bis 2000 Gewichtsprozent.

Die Fasern oder Fasergebilde können einen Schrumpf von 1 bis 60 % aufweisen. Im Fall zweidimensionaler Fasergebilde liegt bevorzugt ein Schrumpf von maximal 50% vor, besonders bevorzugt von maximal 45% und ganz besonders bevorzugt von maximal 35 %.

In einem weiteren Aspekt der Erfindung wird ein Verfahren zur Herstellung von Fasern oder Fasergebilden, ein-, zwei-oder dreidimensional, umfassend Fasern aus einem ersten Faserrohmaterial vorgeschlagen, bei denen die Fasern bei einer vorbestimmten Temperaturtemperatur eine vorbestimmte Temperdauer lang getempert werden. Dabei weist das erste Faserrohmaterial unsubstituierten oder teilweise substituierten Polyvinylalkohol und/oder unsubstituiertes oder teilweise substituiertes Polyvinylalkohol-Copolymer auf.

Vorteilhaft können durch diesen sehr einfachen Prozess Fasern oder Fasergebilde hergestellt werden, die gellerende, insbesondere hydrogelierende Eigenschaften aufweisen. Dabei ist nur ein einziger Prozessschritt zur Stabilisierung der Fasern oder Fasergebilde notwendig, der zudem derart umweltfreundlich ausgebildet ist, dass keinerlei Lösungsmittel, Neben-oder Abfallprodukte anfallen. Zudem können durch das Tempern gegebenenfalls in den Fasern oder Fasergebilde enthaltene Verunreinigungen, wie zum Beispiel Avivage, Spinnhilfsmittel oder Lösungsmittel, entfernt werden.

Man versteht unter Tempern einen Prozess, bei dem eine vorbestimmte Temperatur eine vorbestimmte Zeit lang aufrecht erhalten wird. Werden somit Fasern getempert, so werden die Fasern zuerst auf die vorbestimmte Temperatur gebracht und dann die vorbestimmte Zeit lang auf dieser vorbestimmten Temperatur gehalten. Dabei können auftretende Temperaturschwankungen von zumindest +/-10%, insbesondere +/-5% und bevorzugt +/-1% toleriert werden.

Erfindungsgemäß werden Fasern aus einem ersten Faserrohmaterial mit einem Gewicht von 0,9 bis 10 dtex eingesetzt werden. Bevorzugt werden sie mit einem Gewicht von 1 bis 9 dtex eingesetzt, besonders bevorzugt mit einem Gewicht von 1,5 bis 5 dtex und ganz besonders bevorzugt mit einem Gewicht von 1,9 bis 2,6 dtex.

Die Fasern aus einem ersten Faserrohmaterial können eine Länge von 30 bis 80 mm aufweisen. Bevorzugt werden sie mit einer Länge von 32 bis 70 mm eingesetzt, besonders bevorzugt mit einer Länge von 35 bis 65 mm und ganz besonders bevorzugt mit einer Länge von 38 bis 60 mm.

Wird das Airlaid-Verfahren angewendet, so können die Fasern aus einem ersten Faserrohmaterial eine Länge von 5 bis 20 mm aufweisen. Bevorzugt werden sie dabei mit einer Länge von 6 bis 19 mm eingesetzt, besonders bevorzugt mit einer Länge von 7 bis 18 mm und ganz besonders bevorzugt mit einer Länge von 8 bis 17 mm.

Die vorbestimmte Tempertemperatur kann so gewählt werden, dass sie größer ist als eine Glasübergangstemperatur des verwendeten ersten Faserrohmaterials. Werden mehrere Fasern aus unterschiedlichen Faserrohmaterialien verwendet, so wird die vorbestimmte Tempertemperatur größer als die Glasübergangstemperatur der erfindungsgemäßen Fasern aus Polyvinylalkohol und/oder aus Polyvinylalkohol-Copolymeren gewählt. Zudem kann die vorbestimmte Tempertemperatur derart gewählt werden, dass sie kleiner ist, als eine Schmelztemperatur des verwendeten ersten Faserrohmaterials. Sind dabei mehrere Fasern aus unterschiedlichem Faserrohmaterial verwendet, so wird die vorbestimmte Temperatur so gewählt, dass sie unterhalb der Schmelztemperatur bevorzugt aller verwendeten Faserrohmaterialien liegt.

Erfindungsgemäß liegt die Tempertemperatur in einem Temperaturbereich von 100 bis 200 °C und ganz besonders bevorzugt von 110°C bis 180 °C.

Die vorbestimmte Temperdauer kann von 10 min bis 14h betragen. Bevorzugt beträgt die Temperdauer von 2 bis 10h, besonders bevorzugt von 3 bis 8h und ganz besonders bevorzugt von 4 bis 7h.

Durch die Auswahl derartiger Tempertemperaturen und Temperzeiten kann das erfindungsgemäße Vernetzen der Fasern oder Fasergebilde besonders schonend für die Fasern oder Fasergebilde durchgeführt werden. Zudem ist durch Variation der Tempertemperaturen und Temperzeiten eine unterschiedlich ausgebildete Vernetzung steuerbar, so dass die vernetzten Fasern oder Fasergebilde gegebenenfalls unterschiedliche Eigenschaften aufweisen.

Insbesondere vor oder nach dem Tempern kann ein Verfahren angewendet werden, um ein-, zwei- oder dreidimensionale Fasergebilde zu erhalten. Dabei kann aus den Fasern mittels eines z.B. vorhergehend beschriebenen Verfahrens das jeweilige Fasergebilde hergestellt werden.

Vorteilhaft können so durch ein dementsprechendes Verfestigungsverfahren die Fasern oder Fasergebilde in eine gewünschte Form gebracht werden und in dieser Form verfestigt werden.

Zudem können auch weitere Fasern aus zumindest einem zweiten Faserrohmaterial zugemischt werden.

Vorteilhaft ist die Zumischung von weiteren Fasern nach dem Tempern durchzuführen, wenn die weiteren Fasern beispielsweise durch den Temperprozess leiden und das Tempern der weiteren Fasern zu unerwünschten Ergebnissen führt.

Des Weiteren kann eine Nachbehandlung durchgeführt werden. Zudem ist eine Zumischung von Verarbeitungshilfsstoffen, insbesondere vor dem Verfestigungsverfahren möglich. Ebenfalls eine Zugabe von z.B. zuvor beschriebenen Additiven kann vorgenommen werden.

Als mögliche Nachbehandlung kann eine Nachverfestigung, eine Sterilisierung, eine Bestrahlung, eine Beschichtung, eine Applikation von Avivagen, eine chemische Modifizierung oder eine Weiterverarbeitung vorgenommen werden.

Die einzelnen Verfahrensschritte, Tempern, Verfestigen, Zumischen von weiteren Fasern, Zugabe von Additiven, Zugabe von Verarbeitungshilfsstoffen, und Nachbehandeln können mehrfach in beliebiger Reihenfolge wiederholt werden. Zumindest einmal sollten dabei die Fasern oder die Fasergebilde bei einer vorbestimmten Tempertemperatur eine vorbestimmte Temperdauer lang getempert werden.

Als Verarbeitungshilfsstoffe können Avivage, antistatische Mittel, Tenside, Stabilisatoren, Gleitmittel oder dergleichen zum Einsatz kommen.

In einer bevorzugten Variante des Herstellungsverfahrens werden die Fasern aus einem ersten Faserrohmaterial, insbesondere Polyvinylalkohol-Stapelfasern, zur Vernetzung bei einer vorbestimmten Tempertemperatur, die oberhalb der Glasübergangstemperatur der Fasern aus einem ersten Faserrohmaterial liegt, insbesondere 4 bis 7h lang, getempert. Darauffolgend kann optional eine Zumischung von weiteren Fasern, insbesondere von nicht-gelierenden Fasern, besonders bevorzugt von Polyester-Fasern, mit einem Gewichts-Anteil von 10 bis 50 Gew.% vorgenommen werden. Dann kann aus den so hergestellten Fasern ein zweidimensionales Fasergebilde, wie zum Beispiel ein Vliesstoff, gegebenenfalls unter Einsatz von Verarbeitungshilfsstoffen, wie zum Beispiel Avivage oder antistatischen Mitteln, mittels eines Verfestigungsverfahrens hergestellt werden.

In einer anderen bevorzugten Variante des Herstellungsverfahrens können optional Fasern aus einem ersten Faserrohmaterial mit weiteren Fasern aus einem zweiten Faserrohmaterial vermischt werden, wobei der Anteil an weiteren Fasern bevorzugt 10-50 Gew.% beträgt. Es können aber auch nur Fasern aus einem ersten Faserrohmaterial verwendet werden. Bevorzugt werden als Fasern aus einem ersten Faserrohmaterial Polyvinylalkohol-Fasern und als weitere Fasern aus einem zweiten Faserrohmaterial Polyester-Fasern verwendet. Aus diesen Fasern kann mittels eines Verfestigungsverfahrens ein zweidimensionales Fasergebilde wie zum Beispiel ein Vliesstoff hergestellt werden. Nachfolgend kann das so hergestellte zweidimensionale Fasergebilde bei einer Tempertemperatur oberhalb der Glasübergangstemperatur der Fasern aus einem ersten Faserrohmaterial getempert werden. Optional kann ein derartig hergestelltes zweidimensionales Fasergebilde nachbehandelt werden.

In einem weiteren Aspekt der Erfindung wird die Verwendung von Fasern oder Fasergebilden, wie vorhergehend beschrieben, vorgeschlagen, wobei derartige Fasern oder Fasergebilde zur Herstellung von Materialien für medizinische Anwendungen, insbesondere für Wundauflagen, Wundverbände, Nahtmaterialien, Implantate, Tissue Egineering Scaffolds, Arzneimittel, oder zur Herstellung von Trägermaterialien, Dämmstoffe, Filtermaterialien eingesetzt werden oder bei Herstellung von Hygiene-, Kosmetik,-Haushaltsprodukten, technischen Absorberproduktenk, wie Kabelummantelungen, Produkten für den Lebensmittelbereich, wie Lebensmittelverpackungen, Anwendung finden oder bei der Gewebekonstruktion angewendet werden. Als Hygieneprodukte können unter anderem Damenhygieneprodukte, Windeln und Inkontinenzprodukte verstanden werden. Von den Haushaltsprodukten werden ebenfalls Reinigungsmaterialien umfasst.

Für die jeweilige Verwendung ergeben sich unter anderem die vorhergehend genannten Vorteile.

Als weiterer Aspekt der Erfindung wird ein Wundverband oder eine Wundauflage, enthaltend Fasern oder Fasergebilde wie vorhergehend beschrieben, vorgeschlagen. Derartige Fasern oder Fasergebilde, können bevorzugt im Bereich der Modernen Wundversorgung, insbesondere zur feuchten Wundbehandlung, eingesetzt werden.

Solche Wundverbände oder Wundauflagen können analog klassischer Wundverbände oder Wundauflagen, wie beispielsweise Mullbinden, angewendet werden, weisen aber die vorteilhaften hydrogelierenden Eigenschaften auf, so dass durch erfindungsgemäße Wundverbände oder Wundauflagen ein vorteilhaft verbesserte Wundversorgung erreicht werden kann.

Ausführung der Erfindung

**Beispiel 1: Herstellung von getemperten Fasern aus Polyvinylalkohol**

Polyvinylalkohol-Fasern (2,2 dtex) werden bei 150°C getempert, um eine Vernetzung des Polyvinylalkohols (PVA) zu erzielen.

Ab einer Temperdauer von 2h stellt sich eine Stabilität der PVA-Fasern ein, die sich durch die Ausbildung von stabilen, hydrogelierenden Fasern in 0,9 %iger wässriger Natriumchlorid-Lösung zeigt. Die Stabilität der Fasern steigt mit der Temperdauer. Bei einer Temperdauer von 4 bis 7h weisen die Fasern eine hohe Stabilität auf.

Nach dem Tempern wird die Absorption von 0,9%iger wässriger Natriumchlorid-Lösung der Fasern bestimmt. Die Bestimmung der Absorption erfolgt in Anlehnung an die DIN 53923. Statt der in der DIN 53923 beschriebenen Aufnahme an Wasser wird die Aufnahme an 0,9%iger wässriger Natriumchlorid-Lösung bestimmt. Die Aufnahme an 0,9 %iger wässriger Natriumchlorid-Lösung beträgt abhängig von der Temperdauer und damit des Vernetzungsgrads zwischen 300 bis 3500 Gew.-%.

Die getemperten PVA-Fasern können zu Vliesstoffen weiterverarbeitet werden. Es werden Vliesstoffe aus PVA-Fasern oder aus PVA-Fasern mit Zumischung anderer Fasern wie z.B. Polyester hergestellt. Die aus den getemperten PVA-Fasern hergestellten Vliesstoffe zeigen abhängig von der Faserzumischung und dem Vernetzungsgrad eine hohe Absorption an 0,9%iger wässriger Natriumchlorid-Lösung zwischen 300 bis 3500 Gew.-%.

**Beispiel 2: Nadelverfestigter Vliesstoff aus Polyvinylalkohol**

Es wird ein nadelverfestigter PVA-Vliesstoff hergestellt. Der nadelverfestige Polyvinylalkohol-Vliesstoff aus PVA-Fasern (2,2 dtex) wird bei 150°C getempert, um eine Vernetzung des Polyvinylalkohols zu erzielen.

Ab einer Temperdauer von 2h stellt sich eine Stabilität der PVA-Vliesstoffe ein, die sich durch die Ausbildung von stabilen, hydrogelierenden Vliesen in 0,9 %iger wässriger Natriumchlorid-Lösung zeigt. Die Stabilität der Vliesstoffe steigt mit der Temperdauer. Bei einer Temperdauer von 4 bis 7h weisen die Vliesstoffe eine hohe Stabilität auf.

Nach dem Tempern wird die Absorption von 0,9%iger wässriger Natriumchlorid-Lösung des Vliesstoffes analog Beispiel 1 bestimmt.

Die Aufnahme an 0,9 %iger, wässriger Natriumchlorid-Lösung beträgt abhängig von der Temperdauer und damit des Vernetzungsgrads zwischen 300 bis 2000 Gew.-%.

Zudem wird der Schrumpf der verfestigten Vliesstoffe in 0,9%iger, wässriger Natriumchlorid-Lösung bestimmt. Hierzu werden 10,0 cm × 10,0 cm (Fläche 1) große Vliesstoff-Stücke ausgestanzt und diese in 0,9 %ige wässrige Natriumchlorid-Lösung getaucht. Die Vliesstoffe werden aus der Lösung entnommen und für 2min abgetropft. Danach wird die Größe der Vliesstoffe abgemessen [±1mm] (Fläche 2). Der Schrumpf der Vliese berechnet sich nach folgender Formel:

$$Schrumpf\ [\%] = 100 - \frac{Fläche2\ [cm^2]}{Fläche1\ [cm^2]} * 100$$

Der Schrumpf der Polyvinylalkohol-Vliesstoffe beträgt abhängig von der Temperdauer und damit dem Vernetzungsgrad der Vliesstoffe zwischen 30 und 60%.

**Beispiel 3: Kalander-verfestigter Vliesstoff aus Polyvinylalkohol**

Ein Kalander-verfestigter Polyvinylalkohol-Vliesstoff aus PVA-Fasern(2,2 dtex) wird bei 150°C getempert, um eine Vernetzung des Polyvinylalkohols zu erzielen.

Ab einer Temperdauer von 2h stellt sich eine Stabilität der PVA-Vliesstoffe ein, die sich durch die Ausbildung von stabilen, hydrogelierenden Vliesen in 0,9 %iger wässriger Natriumchlorid-Lösung zeigt. Die Stabilität der Vliesstoffe steigt mit der Temperdauer. Bei einer Temperdauer von 4 bis 7h weisen die Vliesstoffe eine hohe Stabilität auf.

Nach dem Tempern wird die Absorption von 0,9%iger wässriger Natriumchlorid-Lösung des Vliesstoffes analog Beispiel 1 bestimmt.

Die Aufnahme an 0,9 %iger wässriger Natriumchlorid-Lösung beträgt abhängig von der Temperdauer und damit des Vernetzungsgrads zwischen 300 bis 2000 Gew.-%.

Zudem wird der Schrumpf der Vliesstoffe in 0,9%iger, wässriger Natriumchlorid-Lösung analog Beispiel 2 bestimmt. Der Schrumpf der Polyvinylalkohol-Vliesstoffe beträgt abhängig von der Temperdauer und damit dem Vernetzungsgrad der Vliesstoffe zwischen 30 und 60%.

**Beispiel 4: Polyvinylalkohol / Polyester-Mischvliesstoffe**

Es werden nadelverfestigte Mischvliesstoffe aus Polvinylalkohol-Fasern (2,2 dtex) und Polyesterfasern (1,7 dtex) hergestellt. Der Anteil der Polyesterfasern im Mischvliesstoff beträgt zwischen 10 und 50%. Die Vliese werden bei 150°C getempert, um eine Vernetzung der Polyvinylalkohol-Fasern im Vlies zu erzielen.

Ab einer Temperdauer von 2h stellt sich eine Stabilität der Vliesstoffe ein, die sich durch die Ausbildung von stabilen, hydrogelierenden Vliesstoffen in 0,9 %iger, wässriger Natriumchlorid-Lösung zeigt. Die Stabilität der Vliesstoffe steigt mit der Temperdauer. Bei einer Temperdauer von 4 bis 7h weisen die Vliesstoffe eine hohe Stabilität auf.

Nach dem Tempern wird die Absorption von 0,9%iger wässriger Natriumchlorid-Lösung des Vliesstoffes analog Beispiel 1 bestimmt

In Abhängigkeit vom Polyester-Anteil im Vliesstoff beträgt die Absorption an 0,9 %iger wässriger Natriumchlorid-Lösung zwischen 500 und 3000 Gew.-%. Mit steigendem Anteil an Polyester von 10% bis 50% im Vliesstoff erhöht sich die Absorption.

Zudem wird der Schrumpf der verfestigten Vliesstoffe in 0,9%iger, wässriger Natriumchlorid-Lösung analog Beispiel 2 bestimmt.

Der Schrumpf der Vliesstoffe beträgt abhängig vom Polyester-Anteil im Vlies zwischen 1 und 45%. Der Schrumpf der Mischvliesstoffe ist somit deutlich niedriger im Vergleich zu Polyvinylalkohol-Vliesstoffen ohne Polyesterfaser-Zumischung. Mit steigendem Anteil an Polyester im Vliesstoff erniedrigt sich der Schrumpf.

**Beispiel 5: Polyvinylalkohol / Polyester-Mischvliesstoffe**

Es werden nadelverfestigte Misch-Vliesstoffe aus Polvinylalkohol- (2,2 dtex) und Polyesterfasern (3,3 dtex) hergestellt. Der Anteil der Polyesterfasern im Misch-Vliesstoff beträgt zwischen 10 und 50%. Die Vliesstoffe werden bei 150°C getempert, um eine Vernetzung der Polyvinylalkohol-Fasern im Vlies zu erzielen.

Ab einer Temperdauer von 2h stellt sich eine Stabilität der Vliesstoffe ein, die sich durch die Ausbildung von stabilen, hydrogelierenden Vliesstoffen in 0,9 %iger, wässriger Natriumchlorid-Lösung zeigt. Die Stabilität der Vliesstoffe steigt mit der Temperdauer. Bei einer Temperdauer von 4 bis 7h weisen die Vliesstoffe eine hohe Stabilität auf. Nach dem Tempern wird die Absorption von 0,9%iger wässriger Natriumchlorid-Lösung des Vliesstoffes analog Beispiel 1 bestimmt. In Abhängigkeit vom Polyester-Anteil im Vliesstoff beträgt die Absorption an 0,9 %iger wässriger Natriumchlorid-Lösung zwischen 500 und 3500 Gew.-%. Mit steigendem Anteil an Polyester von 10% bis 50% im Vliesstoff erhöht sich die Absorption.

Zudem wird der Schrumpf der verfestigten Vliesstoffe in 0,9%iger, wässriger Natriumchlorid-Lösung analog Beispiel 2 bestimmt

Der Schrumpf der Vliesstoffe beträgt abhängig vom Polyester-Anteil im Vliesstoff zwischen 1 und 45%. Der Schrumpf der Misch-Vliesstoffe ist somit deutlich niedriger im Vergleich zu den Polyvinylalkohol-Vliesen ohne Polyesterfaser-Zumischung. Mit steigendem Anteil an Polyester im Vlies erniedrigt sich der Schrumpf.

### Beispiel 6: Bestimmung der Thermodesorption

Bei der Bestimmung der Thermodesorption werden durch Aufheizen einer Probe von Fasern oder Fasergebilden bei 150°C für 20 min in den Fasern enthaltene organische Komponenten freigesetzt, mittels Kryotrap fokussiert und danach mittels Kaltaufgabesystem in die GC/MS injiziert. Dabei wird ein Thermodesorptionssystem GERSTEL und ein Kaltaufgabesystem KAS GERSTEL verwendet. Mittels GC/MS werden die freigesetzten Komponenten detektiert. Dabei wird ein GC Agilent Technologies 6890N Network GC System, Massenselektiver Detektor Agilent Technologies 5973 verwendet.

Bei nichtgetemperten PVA-Fasern können mittels Thermodesorption Dimethylsulfoxid und Fettalkoholethoxylate, z.B. aus der Avivage, nachgewiesen werden. Nach dem Tempern werden die getemperten PVA-Fasern ebenfalls mittels Thermodesorption untersucht. Es können nach dem Tempern weder Dimethylsulfoxid noch Fettalkoholethoxylate detektiert werden. Durch das Tempern können somit im Vliesstoff oder den Fasern enthaltene Verunreinigungen, wie z.B. Spinnhilfsmittel, Lösungsmittel oder Avivage, entfernt werden.

### Patentansprüche

1. Fasern oder Fasergebilde, ein-, zwei-, oder dreidimensional umfassend Fasern aus einem ersten Faserrohmaterial, wobei das erste Faserrohmaterial unsubstituierten oder teilweise substituierten Polyvinylalkohol und/oder unsubstituiertes oder teilweise substituiertes Polyvinylalkohol-Copolymer enthält, wobei die Fasern oder das Fasergeblide gelierend, insbesondere hydrogelierend, ausgebildet sind, wobei die Fasern durch Tempern bei einer Tempertemperatur in einem Temperaturbereich von 100 bis 200 °C vernetzt sind, **dadurch gekennzeichnet, dass** die Fasern oder Fasergebilde eine Aufnahmekapazität für 0,9%ige, wässrige Natriumchlorid-Lösung, gemessen in Anlehnung an die DIN 53923, von 300 bis 3500 Gew.% aufweisen und die Fasern aus dem ersten Faserrohmaterial ein Gewicht von 0,9 bis 10 dtex aufweisen.

2. Fasern oder Fasergebilde nach Anspruch 1,
   wobei das erste Faserrohmaterial als Polymer-Blend ausgebildet ist und zumindest ein weiteres Polymer aus der folgenden Gruppe aufweisen kann:

   Polyolefine, Polyamide, Polyester, Polyacrylnitrile, Polyvinylchloride, Elastane, Polyvinylchloride, Polyester, Polylactide, Polyglykolide, Polyesteramide, Polycaprolactone, Polyhexamethylenterephthalate, Polyhydroxybutyrate, Polyhydroxyvalerate, Polyvinylamine, Polyvinylacetate, Polyethylenglycole, Polyethylenoxide, Polyvinylpyrrolidone, Polyurethane, Polyacrylate, Cellulose, Cellulosederivate, regenerierte Cellulosen, wie z.B. Viskosen, Celluloseether, wie z.B. Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie z.B. Celluloseacetat, oxidierte Cellulosen, bakterielle Cellulosen, Cellulosecarbonate, Alginate, Chitosane, Gelatinen, Kollagene, Stärken, Hyaluronsäuren, Pektine oder Agar.

3. Fasern oder Fasergebilde nach einem der vorhergehenden Ansprüche, wobei die Fasern oder Fasergebilde zusätzlich weitere Fasern aus zumindest einem zweiten Faserrohmaterial aufweisen, wobei das zweite Faserrohmaterial ausgewählt sein kann aus der Gruppe von folgenden nicht-gelierenden Faserrohmaterialien:

   Polyolefine, Cellulosen, Cellulosederivate, regenerierte Cellulosen wie Viskose, Polyamide, Polyacrylnitrile, Elastane, Polyvinylchloride, tierische Naturfasern, wie z.B. Seide, pflanzliche Naturfasern, wie z.B. Baumwolle, und/oder Polyester
und/oder aus der Gruppe von folgenden gelierenden Faserrohmaterialien:

Alginate, Celluloseether, wie z.B. Carboxymethylcellulosen, Methyl-, Ethylcellulosen, Hydroxymethylcellulosen, Hydroxyethylcellulosen, Hydroxyalkylmethylcellulosen, Hydroxypropylcellulosen, Celluloseester, wie z.B. Celluloseacetat, oxidierte Cellulosen, bakterielle Cellulosen, Cellulosecarbonate, Gelatinen, Kollagene, Stärken, Hyaluronsäuren, Pektine, Agar, Polyvinylamine, Polyvinylacetate, Potyethylenglycole, Polyethylenoxide, Polyvinylpyrrolidone, Polyurethane und/oder Polyacrylate.

4. Fasern oder Fasergebilde nach Anspruch 3,
wobei die weiteren Fasern als Bikomponentenfasern und/oder Mehrkomponentenfasern ausgebildet sind und/oder wobei das zweite Faserrohmaterial als Polymer-Blend ausgebildet ist.

5. Fasern oder Fasergebilde nach Anspruch 3 oder 4,
wobei der Anteil an weiteren Fasern 10-50 Gew.% beträgt.

6. Fasern oder Fasergebilde nach einem der vorhergehenden Ansprüche, wobei die Fasern oder Fasergebilde zusätzlich zumindest ein Additiv ausgewählt aus folgender Gruppe aufweisen:

pharmakologische Wirkstoffe, Medikamente, Antibiotika, Analgetika, Antiinfektiva, entzündungshemmende Mittel, blutstillende Mittel, wundheilungsfördemde Mittel, antimikrobielle Agentien, antibakterielle Agentien, antivirale Agentien, Enzyme, Aminosäuren, Antioxidantien, Peptide, Peptidsequenzen, Polysaccharide, Chitosan, Wachstumsfaktoren, Purine, Pyrimidine, lebende Zellen, $\beta$-Tricalciumphosphat , Hydroxyapatit, insbesondere Hydroxyapatitnanopartikel, geruchsadsorbierende Additive, Cyclodextrine, Metalle, Silber, Gold, Kupfer, Zink, Kohlenstoffverbindungen, Graphit und/oder Verarbeitungshilfsstoffe.

7. Fasergebilde nach einem der vorhergehenden Ansprüche,
wobei ein Schrumpf des jeweiligen zumindest zweidimensionalen Fasergebildes wie folgt gemessen wird:

- es werden *10,0 cm $\times$ 10,0 cm (Fläche1)* große Stücke ausgestanzt und in eine 0,9 %ige wässrige Natriumchlorid-Lösung eingetaucht;
- die ausgestanzten und getränkten Stücke werden aus der Lösung entnommen und für 2min abgetropft;
- danach wird die Größe der Stücke abgemessen (Fläche 2).
- der Schrumpf des Fasergebildes wird dann nach folgender Formel berechnet:

$$Schrumpf\ [\%] = 100 - \frac{Fläche2\ [cm^2]}{Fläche1\ [cm^2]} * 100$$

und wobei der so gemessene Schrumpf maximal 60 % beträgt.

8. Verfahren zur Herstellung von Fasern oder Fasergebilden nach einem der vorhergehenden Ansprüche umfassend das Tempern und Vernetzen von Fasern aus einem ersten Faserrohmaterial, wobei das erste Faserrohmaterial unsubstituierten oder teilweise substituierten Polyvinylalkohol und/oder unsubstituiertes oder teilweise unsubstituiertes Polyvinylalkohol-Copolymer aufweist, bei einer vorbestimmten Tempertemperatur eine vorbestimmte Temperdauer lang.

9. Verfahren nach Anspruch 8,
wobei die vorbestimmte Tempertemperatur größer ist als eine Glasübergangstemperatur und/oder kleiner ist als eine Schmelztemperatur des verwendeten ersten Faserrohmaterials.

10. Verfahren nach einem der Ansprüche 8 oder 9,
wobei die vorbestimmte Temperdauer 10min bis 14h beträgt.

11. Verfahren nach einem der Ansprüche 8 bis 10,
wobei, insbesondere vor oder nach dem Tempern, ein Verfestigungsverfahren zur Herstellung eines ein-, zwei oder dreidimensionalen Fasergebildes angewendet wird.

**12.** Verfahren nach einem der Ansprüche 8 bis 11,
wobei weitere Fasern aus zumindest einem zweiten Faserrohmaterial zugemischt werden.

**13.** Verwendung von Fasern oder Fasergebilden nach einem der Ansprüche 1 bis 7 und/oder hergestellt nach einem Verfahren der Ansprüche 8 bis 12 zur Herstellung von Materialien für medizinische Anwendungen, von Wundauflagen, Wundverbänden, Nahtmaterialien, Implantaten, Tissue Engineering Scaffolds, Arzneimitteln, Trägermaterialien, Dämmstoffen, Filtermaterialien, technische Absorberprodukten, Produkten für den Lebensmittelbereich, Hygieneprodukten, Kosmetikprodukten, Haushaltsprodukten, wobei als Hygieneprodukte Damenhygieneprodukte, Windeln und Inkontinenzprodukte umfasst sind, wobei als Haushaltsprodukte Reinigungsmaterialien umfasst sind.

**14.** Wundverbände oder Wundauflagen enthaltend Fasern oder Fasergebilde nach einem der Ansprüche 1 bis 7 und/oder hergestellt nach einem Verfahren der Ansprüche 8 bis 12.

## Claims

**1.** Fibres or fibrous structures, one-, two- or three-dimensional, comprising fibres from a first fibre raw material, wherein the first fibre raw material contains unsubstituted or partially substituted polyvinyl alcohol and/or unsubstituted or partially substituted polyvinyl alcohol copolymer, wherein the fibres or the fibrous structure are configured to be gelling, in particular hydrogelling, wherein the fibres are crosslinked by heating at an anneal temperature in the temperature range from 100 to 200°C, **characterized in that** the fibres or fibrous structures have an uptake capacity in respect of 0.9% aqueous sodium chloride solution, as measured according to DIN 53923, of 300 to 3500 wt% and the fibres from the first fibre raw material have a weight of 0.9 to 10 dtex.

**2.** Fibres or fibrous structures according to Claim 1, wherein the first fibre raw material is configured as a polymer blend and may include at least one further polymer from the following group:

polyolefins, polyamides, polyesters, polyacrylonitriles, polyvinyl chlorides, elastanes, polyvinyl chlorides, polyesters, polylactides, polyglycolides, polyesteramides, polycaprolactones, polyhexamethylene terephthalates, polyhydroxybutyrates, polyhydroxyvalerates, polyvinylamines, polyvinyl acetates, polyethylene glycols, polyethylene oxides, polyvinylpyrrolidones, polyurethanes, polyacrylates, cellulose, cellulose derivatives, regenerated celluloses, e.g. viscoses, cellulose ethers, e.g. carboxymethylcelluloses, methylethylcelluloses, hydroxymethylcelluloses, hydroxyethylcelluloses, hydroxyalkylmethylcelluloses, hydroxypropylcelluloses, cellulose esters, e.g. cellulose acetate, oxidized celluloses, bacterial celluloses, cellulose carbonates, alginates, chitosans, gelatins, collagens, starches, hyaluronic acids, pectins or agar.

**3.** Fibres or fibrous structures according to either preceding claim,
wherein the fibres or fibrous structures additionally include further fibres from at least one second fibre raw material, wherein the second fibre raw material may be selected from the group of the following non-gelling fibre raw materials:

polyolefins, celluloses, cellulose derivatives, regenerated celluloses such as viscose, polyamides, polyacrylonitriles, elastanes, polyvinyl chlorides, animal-type natural fibres, e.g. silk, vegetable-type natural fibres, e.g. cotton, and/or polyesters and/or from the group of the following gelling fibre raw materials:
alginates, cellulose ethers, e.g. carboxymethylcelluloses, methylethylcelluloses, hydroxymethylcelluloses, hydroxyethylcelluloses, hydroxyalkylmethylcelluloses, hydroxypropylcelluloses, cellulose esters, e.g. cellulose acetate, oxidized celluloses, bacterial celluloses, cellulose carbonates, gelatins, collagens, starches, hyaluronic acids, pectins, agar, polyvinylamines, polyvinyl acetates, polyethylene glycols, polyethylene oxides, polyvinylpyrrolidones, polyurethanes and/or polyacrylates.

**4.** Fibres or fibrous structures according to Claim 3, wherein the further fibres are configured as bicomponent fibres and/or polycomponent fibres and/or wherein the second fibre raw material is configured as a polymer blend.

**5.** Fibres or fibrous structures according to Claim 3 or 4,
wherein the proportion of further fibres is 10-50 wt%.

**6.** Fibres or fibrous structures according to any preceding claim,

wherein the fibres or fibrous structures additionally include at least one additive selected from the following group:

active pharmacological ingredients, medicaments, antibiotics, analgesics, anti-infectives, antiinflammatories, haemostatics, wound healing promoters, antimicrobial agents, antibacterial agents, antiviral agents, enzymes, amino acids, antioxidants, peptides, peptide sequences, polysaccharides, chitosan, growth factors, purins, pyrimidines, living cells, β-tricalcium phosphate, hydroxylapatite, in particular hydroxylapatite nanoparticles, odour-adsorbing additives, cyclodextrins, metals, silver, gold, copper, zinc, carbon compounds, graphite and/or processing aids.

7.  Fibrous structures according to any preceding claim,
    wherein any shrinkage on the part of the particular at least two-dimensional fibrous structure is measured as follows:

    - pieces 10.0 cm × 10.0 cm (area1) in size are stamped out and immersed in a 0.9% aqueous sodium chloride solution;
    - the stamped-out and saturated pieces are removed from the solution and drained for 2 min;
    - thereafter the size of the pieces is measured (area2);
    - the shrinkage of the fibrous structure is then computed according to the following formula:

$$\text{shrinkage}[\%] = 100 - \frac{\text{area2}\,[cm^3]}{\text{area1}\,[cm^3]} \times 100$$

    and wherein the maximum shrinkage thus measured is 60%.

8.  Process for production of fibres or fibrous structures according to any preceding claim, which process comprises heating and crosslinking fibres from a first fibre raw material, wherein the first fibre raw material includes unsubstituted or partially substituted polyvinyl alcohol and/or unsubstituted or partially unsubstituted polyvinyl alcohol copolymer, at a predetermined anneal temperature for a predetermined anneal time.

9.  Process according to Claim 8,
    wherein the predetermined anneal temperature is greater than any glass transition temperature and/or smaller than any melting temperature on the part of the first fibre raw material used.

10. Process according to either of Claims 8 and 9, wherein the predetermined anneal time is in the range from 10 min to 14 h.

11. Process according to any of Claims 8 to 10,
    wherein a consolidating process is applied to produce a one-, two- or three-dimensional fibrous structure, in particular before or after said heating.

12. Process according to any of Claims 8 to 11,
    wherein further fibres from at least one second fibre raw material are admixed.

13. Use of fibres or fibrous structures according to any of Claims 1 to 7 and/or obtained by a process of Claims 8 to 12 in the manufacture of materials for medical applications, of wound contact layers, wound dressings, suture materials, implants, tissue engineering scaffolds, medicinal products, carrier materials, insulants, filter materials, technical absorber products, products for the food sector, hygiene products, cosmetic products, household products, wherein femcare products, diapers and incontinence products are comprehended as hygiene products and cleaning materials are comprehended as household products.

14. Wound dressings or wound contact layers containing fibres or fibrous structures according to any of Claims 1 to 7 and/or obtained by a process of Claims 8 to 12.

**Revendications**

1. Fibres ou structures de fibres, mono-, bi- ou tridimensionnelles, comprenant des fibres d'une première matière première de fibres, la première matière première de fibres contenant de l'alcool polyvinylique non substitué ou partiellement substitué et/ou un copolymère d'alcool polyvinylique non substitué ou partiellement substitué, les fibres ou la structure de fibres étant configurées sous forme gélifiante, notamment hydrogélifiante, les fibres étant réticulées par traitement thermique à une température de traitement thermique dans une plage de température allant de 100 à 200 °C, **caractérisées en ce que** les fibres ou les structures de fibres présentent une capacité d'absorption pour une solution aqueuse de chlorure de sodium à 0,9 %, mesurée selon DIN 53923, de 300 à 3 500 % en poids, et les fibres de la première matière première de fibres présentent un poids de 0,9 à 10 dtex.

2. Fibres ou structures de fibres selon la revendication 1, dans lesquelles la première matière première de fibres est configurée sous la forme d'un mélange de polymères et peut comprendre au moins un polymère supplémentaire du groupe suivant :

   les polyoléfines, les polyamides, les polyesters, les polyacrylonitriles, les polychlorures de vinyle, les élastanes, les polychlorures de vinyle, les polyesters, les polylactides, les polyglycolides, les polyesteramides, les polycaprolactones, les polyhexaméthylène-téréphtalates, les polyhydroxybutyrates, les polyhydroxyvalérates, les polyvinylamines, les polyacétates de vinyle, les polyéthylène glycols, les polyoxydes d'éthylène, les polyvinylpyrrolidones, les polyuréthanes, les polyacrylates, la cellulose, les dérivés de cellulose, les celluloses régénérées, telles que p. ex. les viscoses, les éthers de cellulose, tels que p. ex. les carboxyméthylcelluloses, les méthyl-, éthylcelluloses, les hydroxyméthylcelluloses, les hydroxyéthylcelluloses, les hydroxyalkylméthylcelluloses, les hydroxypropylcelluloses, les esters de cellulose, tels que p. ex. l'acétate de cellulose, les celluloses oxydées, les celluloses bactériennes, les carbonates de cellulose, les alginates, les chitosans, les gélatines, les collagènes, les amidons, les acides hyaluroniques, les pectines ou l'agar-agar.

3. Fibres ou structures de fibres selon l'une quelconque des revendications précédentes, dans lesquelles les fibres ou les structures de fibres comprennent en outre des fibres supplémentaires d'au moins une deuxième matière première de fibres, la deuxième matière première de fibres pouvant être choisie dans le groupe constitué par les matières premières de fibres non gélifiantes suivantes :

   les polyoléfines, les celluloses, les dérivés de cellulose, les celluloses régénérées telles que la viscose, les polyamides, les polyacrylonitriles, les élastanes, les polychlorures de vinyle, les fibres naturelles animales, telles que p. ex. la soie, les fibres naturelles végétales, telles que p. ex. le coton, et/ou les polyesters, et/ou dans le groupe constitué par les matières premières de fibres gélifiantes suivantes :

   les alginates, les éthers de cellulose, tels que p. ex. les carboxyméthylcelluloses, les méthyl-, éthylcelluloses, les hydroxyméthylcelluloses, les hydroxyéthylcelluloses, les hydroxyalkylméthylcelluloses, les hydroxypropylcelluloses, les esters de cellulose, tels que p. ex. l'acétate de cellulose, les celluloses oxydées, les celluloses bactériennes, les carbonates de cellulose, les gélatines, les collagènes, les amidons, les acides hyaluroniques, les pectines, l' agar-agar, les polyvinylamines, les polyacétates de vinyle, les polyéthylène glycols, les polyoxydes d'éthylène, les polyvinylpyrrolidones, les polyuréthanes et/ou les polyacrylates.

4. Fibres ou structures de fibres selon la revendication 3, dans lesquelles les fibres supplémentaires sont configurées sous la forme de fibres bicomposantes et/ou de fibres multicomposantes et/ou dans lesquelles la deuxième matière première de fibres est configurée sous la forme d'un mélange de polymères.

5. Fibres ou structures de fibres selon la revendication 3 ou 4, dans lesquelles la proportion des fibres supplémentaires est de 10 à 50 % en poids.

6. Fibres ou structures de fibres selon l'une quelconque des revendications précédentes, dans lesquelles les fibres ou les structures de fibres comprennent en outre au moins un additif choisi dans le groupe suivant :

   les agents actifs pharmacologiques, les médicaments, les antibiotiques, les analgésiques, les agents anti-infectieux, les agents anti-inflammatoires, les agents hémostatiques, les agents cicatrisants, les agents antimicrobiens, les agents antibactériens, les agents antiviraux, les enzymes, les acides aminés, les antioxydants, les peptides, les séquences peptidiques, les polysaccharides, le chitosan, les facteurs de croissance, les purines, les pyrimidines, les cellules vivantes, le phosphate de tricalcium β, l'hydroxyapatite, notamment les nanoparticules d'hydroxyapatite, les additifs absorbant les odeurs, les cyclodextrines, les métaux, l'argent, l'or, le cuivre, le zinc, les composés carbonés, le graphite et/ou les adjuvants d'usinage.

7. Structures de fibres selon l'une quelconque des revendications précédentes, dans lesquelles un retrait de la structure de fibres à chaque fois au moins bidimensionnelle est mesuré de la manière suivante :

- des pièces d'une taille de 10,0 cm x 10,0 cm (surface 1) sont découpées et immergées dans une solution aqueuse de chlorure de sodium à 0,9 % ;
- les pièces découpées et imprégnées sont sorties de la solution et égouttées pendant 2 minutes ;
- puis la taille des pièces est mesurée (surface 2) ;
- le retrait de la structure de fibres est ensuite calculé par la formule suivante :

$$\text{Retrait } [\%] = 100 - \frac{\text{surface 2 } [cm^2]}{\text{surface 1 } [cm^2]} \times 100$$

et le retrait ainsi mesuré est d'au plus 60 %.

8. Procédé de fabrication de fibres ou de structures de fibres selon l'une quelconque des revendications précédentes, comprenant le traitement thermique et la réticulation de fibres en une première matière première de fibres, la première matière première de fibres comprenant de l'alcool polyvinylique non substitué ou partiellement substitué et/ou un copolymère d'alcool polyvinylique non substitué ou partiellement substitué, à une température de traitement thermique prédéterminée et pendant une durée de traitement thermique prédéterminée.

9. Procédé selon la revendication 8, dans lequel la température de traitement thermique prédéterminée est supérieure à une température de transition vitreuse et/ou inférieure à une température de fusion de la première matière première de fibres utilisée.

10. Procédé selon l'une quelconque des revendications 8 ou 9, dans lequel la durée de traitement thermique prédéterminée est de 10 minutes à 14 h.

11. Procédé selon l'une quelconque des revendications 8 à 10, dans lequel, notamment avant ou après le traitement thermique, un procédé de solidification est appliqué pour la fabrication d'une structure de fibres mono-, bi- ou tridimensionnelle.

12. Procédé selon l'une quelconque des revendications 8 à 11, dans lequel des fibres supplémentaires en au moins une deuxième matière première de fibres sont incorporées.

13. Utilisation de fibres ou de structures de fibres selon l'une quelconque des revendications 1 à 7 et/ou fabriquées par un procédé selon les revendications 8 à 12, pour la fabrication de matériaux pour applications médicales, de compresses, de pansements, de matériaux de suture, d'implants, de supports d'ingénierie tissulaire, de médicaments, de matériaux supports, de matériaux isolants, de matériaux de filtration, de produits absorbants techniques, de produits pour le domaine alimentaire, de produits d'hygiène, de produits cosmétiques, de produits ménagers, les produits d'hygiène comprenant les produits d'hygiène féminine, les couches et les produits pour l'incontinence, et les produits ménagers comprenant les matériaux de nettoyage.

14. Pansements ou compresses contenant des fibres ou des structures de fibres selon l'une quelconque des revendications 1 à 7 et/ou fabriquées par un procédé selon les revendications 8 à 12.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0130407 A1
- WO 2005103097 A1
- EP 0745708 A2

**In der Beschreibung aufgeführte Nicht-Patentliteratur**

- Effect of annealing on aqueous stability and elastic modulus of electrospun poly(vinyl alcohol) fibers. **KENNETH KAR HO WONG et al.** JOURNAL OF MATERIALS SCIENCE. KLUWER ACADEMIC PUBLISHERS, 26. Januar 2010, vol. 45, 2456-2465
- Mechanical Properties and superstructure of Poly(vinyl alcohol)fibers annealed under extremely high tension. **SUZUKI A et al.** Kobunshi Ronbunshu. Society of Polymer Science, 01. Januar 1994, vol. 51, 201-207